(19) Europäisches Patentamt
European Patent Office
Office européen des brevets

(11) EP 0 745 678 B1

(12) **EUROPÄISCHE PATENTSCHRIFT**

(45) Veröffentlichungstag und Bekanntmachung des Hinweises auf die Patenterteilung:
**14.03.2001 Patentblatt 2001/11**

(51) Int Cl.⁷: **C12P 13/04**
// (C12P13/04, C12R1:06)

(21) Anmeldenummer: **96107345.9**

(22) Anmeldetag: **09.05.1996**

(54) **Neuer Mikroorganismus, dessen Verwendung und Verfahren zur Herstellung von L-Alpha-Aminosäuren**

Novel microorganism, its use and process for the preparation of L-alpha-aminoacids

Microorganisme nouveau, son utilisation et procédé pour la préparation des acides L-alpha-aminiques

(84) Benannte Vertragsstaaten:
**CH DE DK ES FI FR GB IE IT LI NL**

(30) Priorität: **30.05.1995 DE 19519717**

(43) Veröffentlichungstag der Anmeldung:
**04.12.1996 Patentblatt 1996/49**

(73) Patentinhaber: **Degussa-Hüls Aktiengesellschaft
60287 Frankfurt am Main (DE)**

(72) Erfinder:
- **Wagner, Fritz, Prof. Dr.
38124 Braunschweig (DE)**
- **Hantke, Britta
38102 Braunschweig (DE)**
- **Wagner, Thomas, Dr.
38124 Braunschweig (DE)**
- **Drauz, Karlheinz, Prof. Dr.
63579 Freigericht (DE)**
- **Bommarius, Andreas, Dr.
60323 Frankfurt (DE)**

(56) Entgegenhaltungen:
**EP-A- 0 159 866** **EP-A- 0 625 571**
**DE-A- 3 942 982**

- **AGARWAL G.P. ET AL.: "Advances in Biochemical Engineering/Biotechnology (Microbial Bioproducts)" 1990 , SPRINGER VERLAG , BERLIN, HEIDELBERG XP002049714 * Seite 31 - Seite 75 ***
- **WAGNER, T. ET AL.: "Production of L-methionine from D,L-5-(2-methylthioethyl)hydantoin by resting cells of a new mutant strain of Arthrobacter species DSM 7330", 1996, Band 46, Seiten 63-68**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 745 678 B1

## Beschreibung

Die Erfindung betrifft einen neuen Mikroorganismus, der bei hohen spezifischen Aktivitäten die Fähigkeit besitzt, D-, oder L-, oder D,L-5-monosubstituierte Hydantoine oder D-, oder L-, oder D,L-N-Carbamoyl-α-Aminosäuren in die korrespondierenden, enantiomerenreinen L-α-Aminosäuren umzusetzen.

Bislang sind zur fermentativen bzw. enzymatischen Umwandlung von N-5-monosubstituierten Hydantoinen in die enantiomerenreinen L-α-Aminosäure zahlreiche Methoden in der Literatur beschrieben oder zum Patent angemeldet worden (s. Syldatk, C., Müller, R., Pietzsch, M., Wagner, F., MICROBIAL AND ENZYMATIC PRODUCTION OF L-AMINO ACIDS FROM D,L-5-MONOSUBSTITUTED HYDANTOINS in: Biocatalytic production of amino acids and derivatives (Rozell, J. D. and Wagner, F., eds.) Hanser Verlag, München, 1992, S. 129 - 176).

Yokozeki, K., Sano, K., Eguchi, C., Iwagami, H. and Mitsugi, K. (1986): OPTIMAL CONDITIONS FOR THE ENZYMATIC PRODUCTION OF L-AROMATIC AMINO ACIDS FROM THE CORRESPONDING 5-SUBSTITUTED HYDANTOINS, Agric. Biol. Chem. 51, 729 - 736 und Yokozeki, K., Hirose, Y. and Kubota, K. (1986): MECHANISM OF ASYMMETRIC PRODUCTION OF L-AROMATIC AMINO ACIDS FROM THE CORRESPONDING HYDANTOINS BY FLAVOBACTERIUM SP., Agric. Biol. Chem., 51, 737 - 746 beschreibt einen Reaktionsmechanismus für die Hydrolyse von 5-Arylalkylhydantoinen am Beispiel von 5-Benzylhydantoin mit einer aufgereinigten Hydantoinase aus Flavobacter sp. AJ-3912. Dabei wurden die relativen Geschwindigkeiten der Hin- und Rückreaktion ermittelt und gezeigt, daß die Hinreaktion von D-5-Benzylhydantoin zum N-Carbamoyl-D-phenylalanin deutlich langsamer erfolgte, als die Hydrolyse von L-5-Benzylhydantoin zum N-Carbamoyl-L-phenylalanin. Über den Mechanismus der enantioselektiven Hydrolyse von 5-Alkylhydantoinen wurde keine Aussage getroffen, da D,L-5-Methylthioethylhydantoin nur in Spuren zu N-Carbamoyl-L-Methionin umgesetzt werden konnte.

Aus der DE 43 16 928 C2 sind die Arthrobacter spec. DSM 7329 und DSM 7330 bekannt, die eine hohe Produktivität aufweisen und vorwiegend aliphatische L-Aminosäuren, wie z. B. L-Methionin aus D-, und/oder L-, und/oder D,L- eines 5-monosubstituierten Hydantoins und/oder der korrespondierenden N-Carbamoyl-aminosäure und/oder einem Gemisch aus den beiden erwähnten Substanzklassen nach dem in Schema 1 wiedergegebenen Reaktionsmechanismus herstellen. "Alkyl" steht hier nur beispielhaft für das o. g. Substratspektrum. Die Pfeilstärke steht für die gefundenen Geschwindigkeiten.

Schema 1

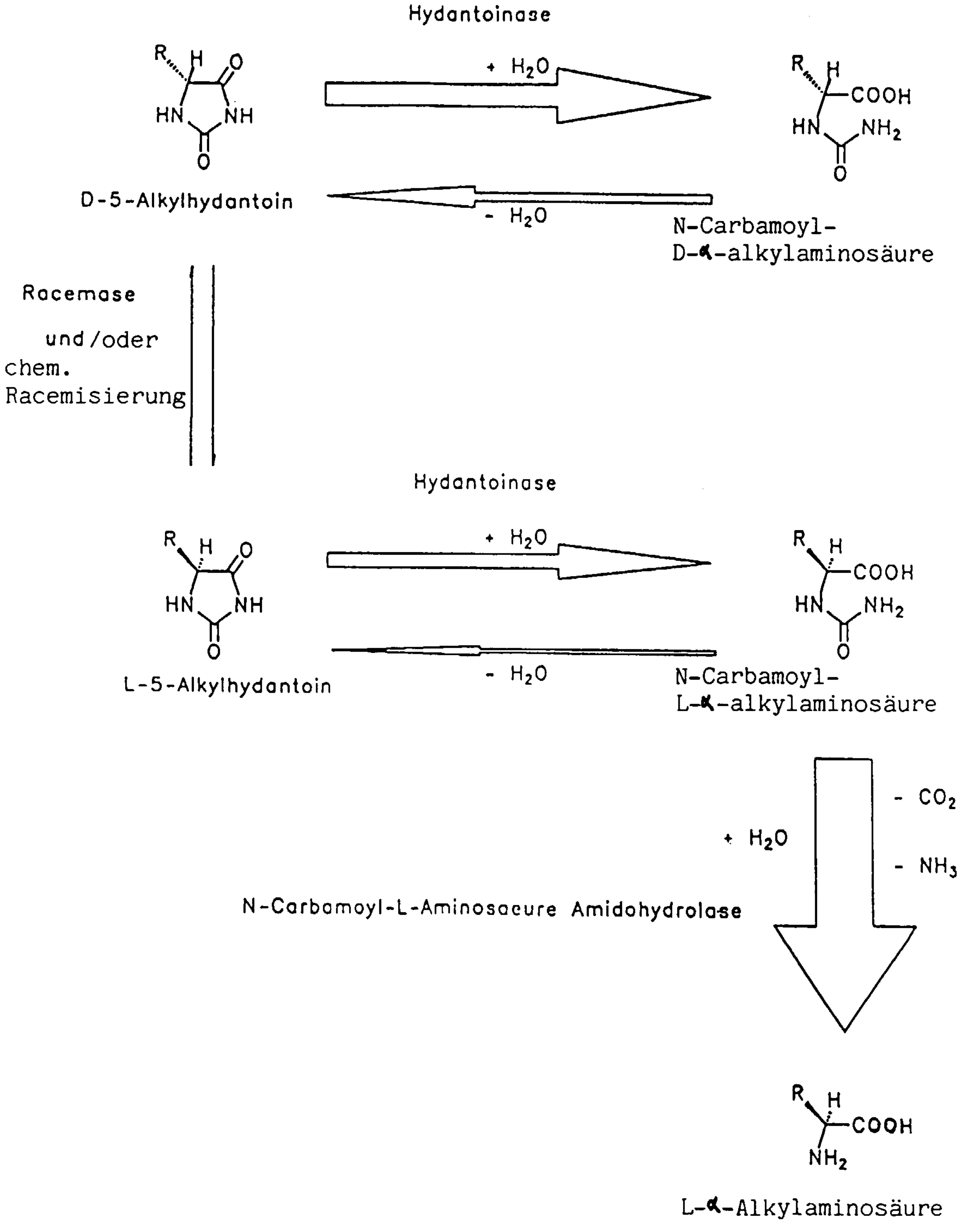


Obwohl die spezifischen Aktivitäten von DSM 7329 und 7330 schon recht beachtlich sind, insbesondere für die Umsetzung von D,L-5-monosubstituierten Hydantoinen über die N-Carbamoyl-α-Aminosäuren zu L-α-Aminosäuren, hat es sich in der Praxis als relativ nachteilig erwiesen, daß produktionstechnisch befriedigende spezifische Aktivitäten nur unter Verwendung eines Induktors erzielbar sind. Hierfür wird bevorzugt 5-Indol-3-ylmethyl-3-methylimidazolidin-2,4-dion (N3-IMH) eingesetzt. Ohne Einsatz dieses Induktors ist offensichtlich die spezifische Aktivität des Hydantoin-spaltenden Enzymsystems in DSM 7329 und 7330 nur relativ gering.

Angesichts des hierin dargelegten Standes der Technik ist es Aufgabe der vorliegenden Erfindung, weitere Mikroorganismen zur Verfügung zu stellen, die einfach zu kultivieren sind und Enzyme herstellen, die in der Lage sind, in größeren Mengen bzw. Geschwindigkeiten L-α-Aminosäuren aus D-, L- und/oder D,L-5-monosubstituierten Hydantoin und/oder einer D-, L- und/oder D,L-N-Carbamoyl-α-aminosäure herzustellen. Aufgabe ist auch ein entsprechendes Verfahren zur Herstellung von L-α-Aminosäuren sowie die Verwendung der Mikroorganismen.

Diese Aufgabe wird mit dem Mikroorganismus DSM 9771 gelöst. Dieser Mikroorganismus wurde am 28.02.1995 bei der DSM - Deutsche Sammlung von Mikroorganismen und Zellkulturen GmbH, Mascheroder Weg 1B, D-38124 Braunschweig, hinterlegt, entsprechende Bescheinigungen liegen der Beschreibung bei.

Bei DSM 9771 handelt es sich um eine aus einer natürlichen Selektion gewonnene Mutante des Stammes Arthrobacter spec. DSM 7330. DSM 9771 weist gegenüber dem Elternorganismus folgende wesentliche Vorteile auf:

- Konstitutive Produktion des Hydantoin-spaltenden Enzymsystems bei erhöhter spezifischer Aktivität gegenüber DSM 7330. Dadurch kann besonders vorteilhaft in einem Produktionsprozeß auf einen sonst notwendigen Induktor wie 5-Indol-3-ylmethyl-3-methyl-imidazolidin-2,4-dion (N3-IMH) verzichtet werden.

- Beschleunigtes Zellwachstum aufgrund des Induktorverzichtes.

- Falls gewünscht kann die spezifische Aktivität des Mikroorganismus durch Induktion mit N3-IMH noch weiter erhöht werden.

- Die Biotransformation von 5-Methylthioethylhydantoin zu L-Methionin mittels ruhender Zellen von DSM 9771 erfolgt stereoselektiv (99.8 % enantiomerenrein).

- Die Umsetzung des Substrates erfolgt in sehr hohen Konzentration innerhalb von 48 h nahezu quantitativ (Ausbeute > 90 %).

Der Stamm DSM 9771 hebt sich von dem Elternorganismus DSM 7330 besonders deutlich dadurch ab, daß er ohne Induktion des Enzymsystems mittels des Induktors 5-Indol-3-ylmethyl-3-methyl-imidazolidin-2,4-dion (N3-IMH) höhere enzymatische Aktivitäten (Faktor 2.3) zeigt. Dies war überraschend und für den Fachmann nicht ohne weiteres vorhersehbar. Insbesondere die Tatsache, daß gerade die gefundene Mutante die hervorragenden Eigenschaften aufweist ist nicht naheliegend gewesen. Der Verzicht des Induktoreinsatzes hat zudem besonders vorteilhaft ein deutlich schnelleres Zellwachstum zur Folge.

Schließlich kann wie bereits erwähnt auch DSM 9771 in Gegenwart eines herkömmlichen Induktors eingesetzt werden. So läßt sich durch Anzucht des Mikroorganismus DSM 9771 in Gegenwart des Induktors N3-IMH beispielsweise die Enzymaktivität um den Faktor 2.7 steigern.

Das Verfahren zur Herstellung von L-α-Aminosäuren durch enzymatische Umsetzung eines D-, oder L-, oder D,L-5-monosubstituierten Hydantoins oder D-, oder L-, oder D,L-N-Carbamoyl-α-Aminosäure wird durchgeführt, indem die Umsetzung mittels des Mikroorganismus DSM 9771 und/oder mittels von diesem Mikroorganismus produzierten Enzymen erfolgt. Aufgrund des neuen Mikroorganismus sowie der stark verbesserten spezifischen Aktivitäten und Ausbeuten während des Verfahrens ist auch das Verfahren der Erfindung in Analogie eines z. B. aus der DE 43 16 928 C2 bekannten Verfahrens nicht naheliegend.

Das Verfahren selbst kann grundsätzlich durchgeführt werden durch:

(a) ruhende oder abgetötete Zellen
(b) eine Maische des Mikroorganismus
(c) einen Rohextrakt
(d) mehr oder weniger aufgereinigte Enzyme des Mikroorganismus.

Wenn das Verfahren mit dem Mikroorganismus selbst durchgeführt wird, dann erfolgt dies am besten mit ruhenden oder abgetöteten Mikroorganismen, damit diese das Substrat nicht selbst verwerten.

Wenn das Verfahren mittels den von dem Mikroorganismus produzierten Enzymen erfolgt, dann kann dies mit einer Maische der Mikroorganismen (z.B. erhalten mittels einer French Press oder Glasperlenmühle), einem Rohextrakt oder mit mehr oder weniger aufgereinigten Enzymen der Mikroorganismen erfolgen.

Das beschriebene Verfahren zur Herstellung von L-α-Aminosäuren verläuft mit dem Mikroorganismus der vorliegenden Erfindung bzw. dessen Enzymen stereospezifisch (99.8 % enantiomerenrein).

Die vorliegende Erfindung betrifft auch die Verwendung des Mikroorganismus DSM 9771 zur Anzucht von

Mutanten oder Varianten dieses Mikroorganismus

oder zur Gewinnung eines eine Carbamoylase und/oder Hydantoinase und/oder Hydantoinracemase codierenden Gens

oder zur Insertion eines eine Carbamoylase und/oder Hydantoinase und/oder Hydantoinracemase codierenden Gens in einen Mikroorganismus oder in eine Zelle

oder zur Herstellung einer L-α-Aminosäure.

Die Anzucht von Mutanten oder Varianten des Mikroorganismus DSM 9771 kann z. B. durch Selektion spontan vorkommender Mutationen erfolgen. Andere Möglichkeiten sind z. B. die Mutationen durch Einwirkung von chemischen Stoffen und/oder radioaktiver Strahlung und/oder UV-Licht.

Die Gewinnung eines oder mehrerer Gene aus dem Mikroorganismus dient z. B. der Sequenzanalyse der Gene für die Enzyme oder zur Insertion in einen Mikroorganismus , wobei durch Auswahl des Mikroorganismus, in den das Gen insertiert wird, ein Mikroorganismus erhalten werden kann, der größere Mengen des oder der für das oben beschriebene Verfahren entscheidenden Enzyme produziert. Gegebenenfalls kann das Gen auch in eine tierische oder pflanzliche Zelle eingebaut werden. Der Einbau des Gens erfolgt mit den in der Mikrobiologie üblichen Methoden, z. B. über einen Vektor.

Der beschriebene neue Mikroorganismus stellt eine Reihe von Enzymen in großen Mengen her, wodurch die Aktivität der Zellmasse für das vorbeschriebene Verfahren extrem hoch ist. Bislang sind keine Stämme mit einer höheren Aktivität für diese Verfahren bekannt geworden.

Das allgemeine Substratspektrum des Mikroorganismus DSM 9771 entspricht dem in der DE 43 16 928 C2 für die Mikroorganismen DSM 7329 und DSM 7330 wiedergegebenen Substratspektrum.

Gemäß dem früher hierin wiedergegebenen Reaktionsschema werden die verschiedensten 5-substituierten Hydantoine zu L-α-Aminosäuren umgesetzt. Gemäß der Erfindung kann der Rest im Hydantoin ein Alkylrest sein, verzweigt sein, cyclisch sein, mit einem oder mehreren Heteroatomen substituiert sein, aromatisch sein, heteroaromatisch sein, etc. Schwierigkeiten bereiten lediglich 5-Methylhydantoin, 5,5-disubstituierte Hydantoine, mehrfach substituierte Phenylhydantoine, wie 5-3'-Methyl-4'-aminomethylphenylhydantoin, sperrige Substituenten unmittelbar am Hydantoin, wie 5-t-Butylhydantoin sowie Reste mit geladenen Substituenten nahe am Hydantoin, wie z. B. 5-β-Carboxylethylhydantoin, 5-γ-Aminopropylhydantoin, 5-δ-Aminobutylhydantoin. Umsetzen ließen sich hingegen eine Vielzahl von Verbindungen, wie z. B. 5-substituierte Hydantoine mit dem Substituenten Isopropyl, n-Propyl, n-Butyl, Methylthioethyl, Isobutyl, Indolylmethyl, Cyclohexylmethyl, Benzyl, Naphthylmethyl, Phenyl, wobei die unterschiedlichsten Substituenten möglich sind, z. B. am Benzyl, p-Fluor, p-Chlor, p-Amino, p-Methoxy, p-Carboxyl. Dem 5-Substituenten im Hydantoin bzw. der Carbamoylaminosäure können somit keine grundsätzlichen Einschränkungen zugesprochen werden, da aufgrund der Vielzahl von möglichen Substituenten und der Vielzahl der möglichen Funktionalisierungen der Substituenten bzw. der Aminosäurereste grundsätzlich alle typischen 5-substituierten Hydantoine oder entsprechende N-Carbamoylaminosäuren als Substrat in Betracht kommen.

Hinsichtlich der Unterscheidung des erfindungesgemäßen DSM 9771 vom DSM 7329 sowie dem Elternorganismus DSM 7330 ergibt sich neben der überragenden spezifischen Aktivität des erfindungsgemäßen Mikroorganismus als weiteres Kriterium zur Unterscheidung die Eigenschaft des DSM 9771 sowohl Glycerin als auch Citronensäure zu verwerten. Weder DSM 7329 noch DSM 7330 waren hierzu in der Lage.

Zur Abgrenzung von anderen bekannten Mikroorganismen wird hiermit auf die DE 43 16 928.7 verwiesen, auf die zu Offenbarungszwecken ausdrücklich Bezug genommen wird. Aufgrund der Offenbarung in der DE 43 16 928.7 ist klar, daß weder DK-200 (EP-A-0 159 866), noch DSM 3745, DSM 3746, DSM 3747 (DE 37 12 539 C2), noch DSM 3306 (DE 37 02 384 A1 entspricht US-A-5,071,752), noch DP-B-1001, DP-B-1002 (japanisches Patent (B2) Hei 4-39316) die Neuheit des vorliegend gefundenen Mikrorganismus in Frage stellen können. Auch die bei Guivarch et al. in Bull. Soc. Chim. Fr. No. 1-2, 1980, S. 91 - 95 beschriebene Bildung von L-Methionin aus D,L-5-Methylthioethylhydantoin mit Arthrobacter ureafaciens beschreibt einen im Mikroorganismus DSM 9771 nicht vorkommenden Reaktionsweg, genauso wie in dem japanischen Dokument Sho 55-29678 und in der japanischen Schrift JP-B-29678/80 Prozesse beschrieben werden, die sich klar vom Verfahren unterscheiden, wie es mit dem erfindungsgemäßen Mikroorganismus DSM 9771 durchgeführt wird.

Wie bereits erwähnt ist DSM 9771 hinsichtlich seiner physiologisch-bakteriologischen Charakterisierung im wesentlichen identisch mit dem Mikroorganismus DSM 7330. Abweichend von dem Patent DE 43 16 928.7 ist der Mikroorganismus DSM 9771 jedoch in der Lage, Glycerin als Kohlenstoffquelle und auch Citronensäure zu verwerten.

Die Berechnung der spezifischen Aktivitäten ganzer Zellen erfolgte nach folgender Gleichung:

$$A\ spez. = \frac{\text{mmol bzw. g umgesetztes Substrat}}{\text{g BFM * h}}$$

DSM 7330 verfügt über folgende spezifische Aktivität für D,L-Methylthioethylhydantoin:

$$A\ spez. = \frac{28.7\ (mmol)}{50\ (g\ BFM)\ *\ 7\ (h)} = 0.082\left(\frac{mmol}{g\ BFM\ *\ h}\right)$$

Unter identischen Bedingungen verfügt der Stamm DSM 9771 über folgende spezifische Aktivität für D,L-Methylthioethylhydantoin:

$$A\ spez. = \frac{28.7\ (mmol)}{50\ (g\ BFM)\ *\ 3\ (h)} = 0.191\left(\frac{mmol}{g\ BFM\ *\ h}\right)$$

d. h. eine Steigerung der spezifischen Aktivität für D,L-MTEH um den Faktor 2.3. Unter Verwendung höherer Substratkonzentrationen und doppelter Zellfeuchtmasse wurde eine spezifische Aktivität von:

$$A\ spez. = \frac{86.1\ (mmol)}{100\ (g\ BFM)\ *\ 2\ (h)} = 0.43\left(\frac{mmol}{g\ BFM\ *\ h}\right)$$

ermittelt, d. h. eine weitere Steigerung um den Faktor 2.3. Unter Verwendung während der Kultivierung mittels N3-IMH induzierter Zellfeuchtmasse wurde folgende spezifische Aktivität ermittelt:

$$A\ spez. = \frac{86.1\ (mmol)}{100\ (g\ BFM)\ *\ 0.75\ (h)} = 1.148\left(\frac{mmol}{g\ BFM\ *\ h}\right)$$

d. h. eine weitere Steigerung um den Faktor 2.7, bzw. bezogen auf DSM 7330 um den Faktor 14.

Beispiel 1: Gewinnung der Mutante

Durch Kultivierung des Wildstammes DSM 7330 unter Selektionsdruck konnte die Mutante DSM 9771 gewonnen werden. Der Selektionsdruck wurde durch L-Carbamoylmethionin (L-CAM) als einzige Stickstoffquelle erzeugt. Die Kultivierung erfolgte in 500 ml Schüttelkolben mit 100 ml Selektionsmedium a 3d bei 30 °C über 10 Zyklen. Als Inokulum für die Startkultur dienten 10 mg BFM vom DSM 7330. Für die folgenden Kultivierungen wurden jeweils 5 ml der vorherigen Kultur eingesetzt.

| Selektionsmedium: | |
|---|---|
| Glucose | 10.0 g/l |
| $KH_2PO_4$ | 0.95 g/l |
| $K_2HPO_4*3H_2O$ | 2.0 g/l |
| $MgSO_4*7H_2O$ | 0.2 g/l |
| $CaCl_2*2H_2O$ | 0.02 g/l |
| Spurensalzlsg. | 10.0 ml/l |
| pH 7.0 | |
| L-CAM | 2.0 g/l (als sterilfiltrierte Lösung addiert) |

| Spurensalzlösung: | |
|---|---|
| Citronensäure*$H_2O$ | 150 mg/l |
| $H_3BO_4$ | 50 mg/l |
| $CuSO_4$*5$H_2O$ | 4 mg/l |
| $FeCl_3$*6$H_2O$ | 20 mg/l |
| $MnSO_4$*7$H_2O$ | 40 mg/l |
| $ZnSO_4$*7$H_2O$ | 40 mg/l |
| KJ | 10 mg/l |
| Ammoniumheptamolybdat | 20 mg/l |

Nach der 10. Kultivierung wurde zur Isolierung von Einzelkolonien ein Verdünnungsausstrich auf Agarplatten mit Selektionsmedium durchgeführt und diese 4d bei 30 °C inkubiert. Die vereinzelten Kolonien wurden gepickt, auf Schrägagarröhrchen mit Komplexmedium 1 angezogen und bei 4 °C gelagert. Zur Gewinnung von Biomasse für die folgenden Aktivitätstests wurden die Klone 14 - 16 h bei 30 °C im Komplexmedium 2 kultiviert.

Das Komplexmedium 1 setzt sich wie folgt zusammen:

| | |
|---|---|
| Techn. Hefeextrakt | 10.0 g/l |
| Bacto-Pepton | 10.0 g/l |
| Glucose | 10.0 g/l |
| NaCl | 3.0 g/l |
| $MgCl_2$*4$H_2O$ | 0.1 g/l |
| Spurensalzlösung | 10.0 ml/l |
| Agar | 15.0 g/l |
| pH | 7.0 |

Komplexmedium 2 setzt sich wie folgt zusammen:

| | |
|---|---|
| Techn. Hefeextrakt | 1.0 g/l |
| Glucose | 10 g/l wird steril als Lsg. zupipettiert |
| $K_2HPO_4$*3$H_2O$ | 7.61 g/l wird steril als Lsg. zupipettiert |
| $KH_2PO_4$ | 4.54 g/l wird steril als Lsg. zupipettiert |
| $(NH_4)_2SO_4$ | 6.5 g/l |
| Citronensäure*$H_2O$ | 0.32 g/l |
| $MgSO_4$*7$H_2O$ | 0.20 g/l |
| $CaCl_2$*2$H_2O$ | 0.02 g/l |
| $MnCl_2$*4$H_2O$ | 0.02 g/l |
| $FeSO_4$*7$H_2O$ | 0.02 g/l |
| pH | 6.8 |

Die enzymatische Aktivität der so erhaltenen Zellfeuchtmasse (BFM) wird unter folgenden Reaktionsbedingungen vergleichend getestet: 800 µl 0.1 M Tris-HCl-Puffer pH 8.5, werden mit 1 mg D,L-5-Methylthioethylhydantoin (D,L-MTEH) und 200 µl einer 10 %igen Zellsuspension versetzt und 3 h bei 37 °C kultiviert. Danach wird die Zellsuspension zentrifugiert und der klare Überstand mittels HPLC-Analytik untersucht.

Als besonders aktiv hat sich ein Klon herausgestellt, der bei der Deutschen Sammlung für Mikroorganismen (DSM) hinterlegt wird. Eine Klassifizierung des Stammes ergab, daß es sich um eine neue Art der Arthrobactergruppe handelt.

Beispiele für die Umsetzungen von D,L-5-monosubstituierten Hydantoinen, die mit dem erfindungsgemäßen Mikroorganismus über die N-Carbamoyl-α-Aminosäuren zu L-α-Aminosäuren entsprechend dem gezeigten Reaktionsmechanismus hydrolysiert werden können, sind in Tabelle 1 aufgeführt.

Tabelle 1:

| R = | Einsatz [mmol] | D,L-Hydantoin [mmol] | N-Carbamoyl-derivat [mmol] | L-α-Amino-säure [mmol] |
|---|---|---|---|---|
| 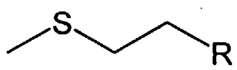<br>Methylthioethyl- | 28.7 | **6.60**<br>11.09 | **11.01**<br>11.7 | **10.74**<br>5.48 |
| 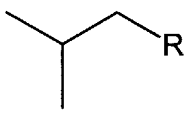<br>iso-Butyl- | 32 | **n. b.**<br>n. b. | **n. b.**<br>n. b. | **+**<br>+ |
| 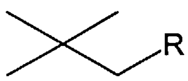<br>tert-Butyl- | 32 | **n. b.**<br>n. b. | **n. b.**<br>n. b. | **-**<br>- |
| 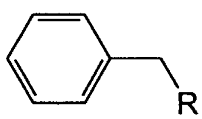<br>Benzyl- | 26.3 | **-**<br>- | **2.0**<br>4.82 | **27.19**<br>22.94 |
| 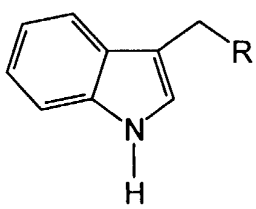<br>Indolylmethyl- | 20.6 | **-**<br>- | **0.34**<br>2.41 | **20.32**<br>18.15 |
| 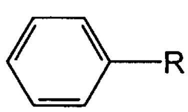<br>Phenyl- | 28.38 | **8.5**<br>22.43 | **n. b.**<br>n. b. | **n. b.**<br>n. b. |

Reaktionsbedingungen: Die angegebene Substratkonzentration wird 10 ml = 1 M TRIS-HCl-Puffer pH 8.5, mit 0.5 g BFM über 4 h bei 37 °C inkubiert.
Fett gedruckt dargestellt: induzierte Zellen, n. b.: nicht bestimmbar, +: nachweisbar, -: nicht nachweisbar.

Beispiel 2: Anzucht der Biomasse

Vier 500 ml Erlenmeyerkolben mit 100 ml Komplexmedium 1 (s. o.) wurde mit 10 mg BFM des Stammes DSM 9771 von einer frischen Schrägagarkultur beimpft und 23 h bei 30 °C und 100 UpM kultiviert. Zehn 2000 ml Erlenmeyerkolben mit je 500 ml Komplexmedium 3 werden mit je 25 ml der 1. Vorkultur angeimpft und 14 h bei 30 °C und 100 UpM inkubiert. Nach der Inkubationszeit dient diese Vorkultur II als Inokulum für einen 50 l Bioreaktor. Dieser ist ausgerüstet mit einem Intensorrührer und wird mit 45 l Komplexmedium 4 versetzt, bei pH 6.8 und einer Temperatur von

121 °C und ca. 1 bar Überdruck 30 min sterilisiert. Optional wird das Nährmedium mit 0.5 g/l N3-IMH versetzt. Nach Abkühlen auf 30 °C wird mit 10 %iger NaOH auf pH 7.0 eingestellt und anschließend mit der Suspensionskultur aus Vorkultur II angeimpft. Die Züchtung erfolgt bei 30 °C und einer Rührerdrehzahl von 400 UpM und einer Belüftungsrate von 0.8 V/V/m. Nach 11.5 h bzw. 15 h für den Fall der induzierten Kultivierung wird die Zellsuspension abzentrifugiert. Die so erhaltene Zellfeuchtmasse kann sofort oder nach Zwischenlagerung bei -18 °C zu einem späteren Zeitpunkt für die enzymatische Umsetzung verwendet werden. Es werden 2.8 kg bzw. 2.6 kg (Induktion) Zellfeuchtmasse erhalten.

| Komplexmedium 3: | |
|---|---|
| Techn. Hefeextrakt | 0.75 g/l |
| Glucose | 10 g/l wird steril als Lsg. zupipettiert |
| $K_2HPO_4*3H_2O$ | 7.61 g/l wird steril als Lsg. zupipettiert |
| $KH_2PO_4$ | 4.54 g/l wird steril als Lsg.zupipettiert |
| $(NH_4)_2SO_4$ | 4.0 g/l |
| Citronensäure*$H_2O$ | 0.32 g/l |
| $MgSO_4*7H_2O$ | 0.20 g/l |
| $CaCl_2*2H_2O$ | 0.02 g/l |
| $MnCl_2*4H_2O$ | 0.02 g/l |
| $FeSO_4*7H_2O$ | 0.02 g/l |
| pH | 6.8 |

| Komplexmedium 4: | |
|---|---|
| Techn. Hefeextrakt | 0.75 g/l |
| Glucose | 20 g/l wird steril als Lsg. zupipettiert |
| $K_2HPO_4*3H_2O$ | 0.761g/l wird steril als Lsg. zupipettiert |
| KH2P04 | 0.454 g/l wird steril als Lsg. zupipettiert |
| $(NH_4)_2SO_4$ | 4.0 g/l |
| Citronensäure*$H_2O$ | 0.64 g/l |
| $MgSO_4*7H_2O$ | 0.40 g/l |
| $CaCl_2*2H_2O$ | 0.04 g/l |
| $MnCl_2*4H_2O$ | 0.04 g/l |
| $FeSO_4*7H_2O$ | 0.04 g/l |
| pH | 6.8 |

Beispiel 3:

15 g nicht induzierte Zellfeuchtmasse von DSM 9771 und 2.25 g D,L-MTEH (13 mmol) werden in 150 ml Saline resuspendiert und bei 37 °C, pH 8.5, unter $N_2$-Atmosphäre und pH-statischer Reaktionsführung in einem 250 ml Rührreaktor 30 h inkubiert. Anschließend werden die Zellen abzentrifugiert und der Überstand mittels Hochdruckflüssigkeitschromatographie (HPLC) analysiert. Im Überstand werden 1.6 g (10.74 mmol) L-Methionin nachgewiesen.
Ausbeute = 82.6 % d. Th.

Beispiel 4:

15 g induzierte Zellfeuchtmasse von DSM 9771 und 2.25 g D,L-MTEH (13 mmol) werden in 150 ml Saline resuspendiert und bei
37 °C, pH 8.5, unter $N_2$-Atmosphäre und pH-statischer Reaktionsführung in einem 250 ml Rührreaktor 30 h inkubiert. Anschließend werden die Zellen abzentrifugiert und der Überstand mittels Hochdruckflüssigkeitschromatographie (HPLC) analysiert. Im Überstand werden 1.7 g (11.4 mmol) L-Methionin nachgewiesen.
Ausbeute = 87.8 % d. Th.

Beispiel 5:

Bestimmung des pH-Optimums

Zur Bestimmung des pH-Optimums von DSM 9771 für die Biotransformation von D,L-MTEH zu L-Methionin wurden Umsetzungen mit dem Glycylglycin-Piperazin-Puffer in einem pH-Bereich von 5.0 bis 10.5 durchgeführt. Der Assay wurde in 100 ml Schüttelkolben mit zwei Schikanen durchgeführt. Vorgelegt wurden 9 ml 0.1 M Glycylglycin-Piperazin-Puffer, in dem bereits 1 g/l D,L-MTEH gelöst waren. Als Biokatalysator wurde 1 ml 50 %ige BFM-Saline-Suspension des DSM 9771 eingesetzt und inkubiert wurde unter $N_2$-Atmosphäre bei 37 °C über 3.5 h im Schüttelwasserbad.

Bereits nach 1 h war das Substrat im pH-Bereich zwischen 8.0 und 9.0 vollständig abgebaut. Die höchste Met-Konzentration wurde dagegen bei pH 8.0 gemessen. Dieses Optimum verschiebt sich nach 2 h Inkubation auf den pH-Bereich von 7.0 bis 8.0, bei dem ebenfalls das Substrat vollständig abgebaut ist. Die folgenden Umsetzungen wurden bei einem pH-Wert von 7.5 durchgeführt, weil hierbei die Bildung der L-Aminosäure begünstigt wird.

Beispiel 6:

Bestimmung des Temperaturoptimums

Die Untersuchungen zur Ermittlung der optimalen Temperatur für die Hydrolyse von D,L-MTEH zu L-Met mit dem DSM 9771 als Biokatalysator wurden ebenfalls in 100 ml Schüttelkolben durchgeführt. Als Substratlösung wurden 5 g/l D,L-MTEH in 0.1 M Tris-HCl-Puffer pH 7.5 eingesetzt. Pro Temperatur (25 °C, 30 °C, 34 °C, 37 °C, 40 °C, 45 °C und 50 °C) wurden zwei Kolben mit Puffer-Substratlösung 30 min vorinkubiert und anschließend wurde die Reaktion mit 1 ml 50 %iger BFM-Saline-Suspension gestartet. Für jede Temperatur wurde eine Kinetik über 5 h aufgenommen. Nach 2 h Inkubation konnte für die oben beschriebene Reaktion ein Temperaturoptimum von 34 - 37 °C ermittelt werden, dies entspricht dem Temperaturoptimum des Wildstammes DSM 7330.

Beispiel 7:

140 g induzierte Zellfeuchtmasse von DSM 9771 und 42 g D,L-MTEH (24 mmol) werden in 1400 ml Saline resuspendiert und bei 37 °C unter $N_2$-Atmosphäre und pH-statischer Reaktionsführung in einem 1500 ml Rührreaktor für 8.5 h inkubiert. Nach Zentrifugation werden im Überstand 32.8 g (220 mmol) L-Methionin nachgewiesen.
Ausbeute = 91 % d. Th.

Beispiel 8:

140 g induzierte Zellfeuchtmasse von DSM 9771 und 42 g D,L-MTEH (241 mmol) werden in 1400 ml Saline resuspendiert und bei 34 °C unter $N_2$-Atmosphäre und pH-statischer Reaktionsführung in einem 1500 ml Rührreaktor inkubiert. Nach jeweils 1.5 h werden 14 g (80.4 mmol) D,L-MTEH 9 mal zudosiert. Nach 48 h wird die Reaktion abgebrochen. Da das Produkt L-Methionin in kristalliner Form vorliegt, wird die Reaktionslösung mit dem dreifachen Volumen Saline verdünnt. Anschließend werden die Zellen abzentrifugiert und der Überstand mittels Hochdruckflüssigkeitschromatographie (HPLC) analysiert. Im Überstand werden 142 g (952 mmol) L-Methionin nachgewiesen.
Ausbeute = 98.8 % d. Th.

## Patentansprüche

1. Mikroorganismus DSM 9771.

2. Verfahren zur Herstellung von L-$\alpha$-Aminosäuren durch enzymatische Umsetzung eines D-, L- und/oder D,L-5-monosubstituierten Hydantoins,
**dadurch gekennzeichnet,**
daß die Umsetzung mittels des Mikroorganismus DSM 9771 und/oder mittels von diesem Mikroorganismus produzierten Enzymen erfolgt.

3. Verfahren zur Herstellung von L-$\alpha$-Aminosäuren durch enzymatische Umsetzung einer D-, L- und/oder D,L-N-Carbamoyl-$\alpha$-aminosäure,
**dadurch gekennzeichnet,**

daß die Umsetzung mittels des Mikroorganismus DSM 9771 und/oder mittels von diesem Mikroorganismus produzierten Enzymen erfolgt.

**4.** Verfahren nach Anspruch 2 und/oder 3,
**dadurch gekennzeichnet,**
daß die Umsetzung mit ruhenden Mikroorganismen erfolgt.

**5.** Verwendung des Mikroorganismus DSM 9771 zur Anzucht von Mutanten oder Varianten dieses Mikroorganismus.

**6.** Verwendung des Mikroorganismus DSM 9771
zur Gewinnung eines eine Carbamoylase und/oder Hydantoinase und/oder Hydantoinracemase codierenden Gens.

**7.** Verwendung des Mikroorganismus DSM 9771
zur Insertion eines eine Carbamoylase und/oder Hydantoinase und/oder Hydantoinracemase codierenden Gens in einen Mikroorganismus oder in eine Zelle.

**8.** Verwendung des Mikroorganismus DSM 9771 zur Anzucht von Mutanten oder Varianten dieses Mikroorganismus zur Herstellung einer L-α-Aminosäure.

**Claims**

**1.** Microorganism DSM 9771.

**2.** Process for the production of L-α-amino acids by enzymatic conversion of a D-, L-, and/or D,L-5-mono-substituted hydantoin,
characterised in that
the conversion proceeds by means of microorganism DSM 9771 and/or by means of enzymes produced by this microorganism.

**3.** Process for the production of L-α-amino acids by enzymatic conversion of a D-, L-, and/or D,L-N-carbamoyl-α-amino acid,
characterised in that
the conversion proceeds by means of microorganism DSM 9771 and/or by means of enzymes produced by this microorganism.

**4.** Process according to claims 2 and/or 3
characterised in that
the conversion is performed with dormant microorganisms.

**5.** Use of the microorganism DSM 9771 for cultivating mutants or variants of this microorganism.

**6.** Use of the microorganism DSM 9771 for obtaining a gene coding a carbamoylase and/or hydantoinase and/or hydantoinracemase.

**7.** Use of the microorganism DSM 9771 for inserting a gene coding a carbamoylase and/or hydantoinase and/or hydantoinracemase into a microorganism or cell.

**8.** Use of the microorganism DSM 9771 for the cultivation of mutants or variants of this microorganism for the production of an L-α-amino acid.

**Revendications**

**1.** Microoroganisme DSM 9771.

**2.** Procédé de production d'acides L-α-aminés par réaction enzymatique d'une hydantoïne D-, L- et/ou D,L-5-monosubstituée,
caractérisé en ce qu'
on conduit la réaction au moyen du micro-organisme DSM 9771 et/ou au moyen d'enzymes produites par ce micro-organisme.

**3.** Procédé de préparation d'acides L-$\alpha$-aminés par réaction enzymatique d'un acide D-, L- et/ou D,L-N-carbamoyl-$\alpha$-aminé,
caractérisé en ce qu'
on conduit la réaction au moyen du micro-organisme DSM 9771 et/ou au moyen d'enzymes produites par ce micro-organisme.

**4.** Procédé selon la revendication 2 et/ou 3,
caractérisé en ce qu'
on conduit la réaction avec des micro-organismes au repos.

**5.** Utilisation du micro-organisme DSM 9771 pour la culture de mutants ou de variantes de ce micro-organisme.

**6.** Utilisation du micro-organisme DSM 9771 pour l'obtention d'un gène codant pour une carbamoylase et/ou une hydantoïnase et/ou une racémase d'hydantoïne.

**7.** Utilisation du micro-organisme DSM 9771 pour l'insertion d'un gène codant pour une carbamoylase et/ou une hydantoïnase et/ou une hydantoïne-racémase dans un micro-organisme ou dans une cellule.

**8.** Utilisation du micro-organisme DSM 9771 pour la culture de mutants ou de variantes de ce micro-organisme en vue de la production d'un acide L-$\alpha$-aminé.